(19) Europäisches Patentamt
European Patent Office
Office européen des brevets

(11) EP 4 389 116 B1

(12) EUROPEAN PATENT SPECIFICATION

(45) Date of publication and mention of the grant of the patent:
**10.09.2025 Bulletin 2025/37**

(21) Application number: **23151010.8**

(22) Date of filing: **10.01.2023**

(51) International Patent Classification (IPC):
***A61K 9/10*** *(2006.01)* ***A61K 47/10*** *(2017.01)*
***A61P 33/00*** *(2006.01)* ***A61P 33/10*** *(2006.01)*
***A61K 31/4184*** *(2006.01)*

(52) Cooperative Patent Classification (CPC):
**A61K 9/10; A61K 9/0056; A61K 31/4184; A61K 47/10; A61P 33/00; A61P 33/10;** A61K 9/146

(54) **PHARMACEUTICAL AQUEOUS FLUBENDAZOLE SUSPENSION**

PHARMAZEUTISCHE WÄSSRIGE FLUBENDAZOLSUSPENSION

SUSPENSION PHARMACEUTIQUE AQUEUSE DE FLUBENDAZOLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.12.2022 EP 22216470**

(43) Date of publication of application:
**26.06.2024 Bulletin 2024/26**

(73) Proprietor: **Dechra Ltd**
**Skipton North Yorkshire BD23 2RW (GB)**

(72) Inventors:
- **MICHIELS, Henricus Fransiscus Catharina**
  **BLADEL (NL)**
- **FOLMER, Brigitte Johanna Bernita**
  **BLADEL (NL)**
- **LEJEUNE, Stephan Jacob**
  **BLADEL (NL)**

(74) Representative: **Algemeen Octrooi- en Merkenbureau B.V.**
**P.O. Box 645**
**5600 AP Eindhoven (NL)**

(56) References cited:
**BR-A2- 102018 017 054**
**US-A1- 2014 255 498**
**US-B2- 8 777 134**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

The present invention relates to pharmaceutical aqueous suspensions comprising flubendazole and water. The present invention moreover relates to a (concentrated) medicated water comprising the pharmaceutical aqueous suspension and water. The present invention in addition relates to a process of preparing the pharmaceutical aqueous suspension and to uses of said pharmaceutical aqueous suspension in drinking water for animals.

### Background

Parasitic infections remain an important problem in livestock rearing. Therapeutic use of anthelmintic drugs has been and still is a standard practice for every farm, especially where pigs or poultry species are reared.

Flubendazole has been used for many years as an anthelmintic drug; it has a broad spectrum activity and is very potent against different stages of worms with a good safety margin. For livestock it is advantageous to provide the drug to the target animal by means of its feed (e.g. as a top dressing or mixed in the feed) or via the water supply. The latter has the advantage of increased flexibility as compared to either parenteral or in-feed administration. However, the administration via water is also difficult since flubendazole is a water-insoluble drug. Veterinary formulations for administration of water-insoluble drugs to a target animal through a water distribution system are known. However, the stability of these formulations, especially shelf life and stability after mixing with the drinking water - are not always sufficient. Such stability is required to provide an even distribution of the drug to the target animals, to prevent any clogging in water delivery systems, cups, nipples etc. and to ensure a long shelf life. In order to achieve precise dosing, a high homogeneity of the medicated water is required.

Watering systems are usually complex, comprising of a series of tanks, hundreds of meters of pipes and hundreds of individual nipples or cups. Sedimentation of solid drug particles during the transport of said medicated water might cause serious harm to the system and decreases the dosing precision which might lead to underdosing, which might lead to resistance.

WO2007144362A1 discloses a pharmaceutical aqueous suspension for drinking water administration comprising benzimidazole carbamate particles having an effective average particle size of less than about 450 nm. It further specifies the need for a specific type of surfactant, being a Tween-type surfactant, e.g. polysorbate 80. Patent document BR102018017054 discloses an aqueous suspension comprising nano-sized flubendazole.

The present invention aims at providing a novel aqueous pharmaceutical aqueous suspension of flubendazole that provides excellent physical stability both in the suspension as such as in the final medicated water prepared therewith. The present invention also aims at providing a novel aqueous suspension of flubendazole that provides excellent chemical stability in the suspension. In addition, it aims at providing a novel aqueous suspension of flubendazole that provides an excellent physical stability in the medicated water that may be prepared using the aqueous suspension. The present invention addresses the above problem of stability.

### STATEMENT OF THE INVENTION

In one aspect, the invention relates to a pharmaceutical aqueous suspension comprising: at most 35 wt.% of flubendazole, based on the weight of the pharmaceutical aqueous suspension, as flubendazole solid particles having a particle size distribution in which D50 $\leq$ 0.45 micrometer and $\geq$ 90 % of particles are $\leq$ 1.0 micrometer; a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25, one or more preservatives, and water.

In another aspect, the invention relates to a medicated water comprising water and the pharmaceutical aqueous suspension according to the invention in an amount of between 0.1 ml and 1.5 ml per liter of water. In another aspect, the invention relates to a concentrated medicated water comprising water and the pharmaceutical aqueous suspension according to the invention in an amount of between 50 and 300 ml, such as 150 ml, per liter of water. In yet another aspect, the invention relates to a process of preparing a pharmaceutical aqueous suspension, comprising the steps of: preparing a mixture of solid flubendazole particles, a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25, and water; milling said mixture so obtain an aqueous suspension; and adding one or more preservatives to said aqueous suspension; in order to obtain a pharmaceutical aqueous suspension composition comprising at most 35 wt.% of flubendazole, based on the weight of the pharmaceutical aqueous suspension, as solid

particles having a particle size distribution in which D50 ≤ 0.45 micrometer and ≥ 90 % of particles are ≤ 1.0 micrometer.

In another aspect, the invention relates to a pharmaceutical aqueous suspension for use in the treatment of internal parasites in mammals, such as poultry and pigs or for the preparation of a medicated water to provide a dosage of between 1 and 2.5 mg of flubendazole per kg of body weight per day.

Below several preferred features are disclosed. These features are applicable to the pharmaceutical aqueous suspension as well as to the (concentrated) medicated water, the process and the composition for use.

## DETAILED DESCRIPTION

The present invention is elucidated below with a detailed description. When used in these specification and claims, the terms "comprises" and "comprising" and variations thereof mean that the specified features, steps or integers are included. The terms are not to be interpreted to exclude the presence of other features, steps or components.

### Pharmaceutical aqueous suspension

The pharmaceutical aqueous suspension comprises an aqueous suspension of flubendazole in water. In the present description, the word "solvent" is used as having the meaning of solvent as well as having the meaning of suspending agent in a suspension. Water is considered to be the main solvent or suspending agent in the pharmaceutical aqueous suspensions according to the present invention. In the present description, "pharmaceutical aqueous suspension" or "pharmaceutical suspension" or "suspension" means the subject of the first aspect; this is the pharmaceutical product that is to be marketed as such. In the present description, "medicated water" means a dilution of the pharmaceutical aqueous suspension such that it can be consumed by animals. In the present description, "concentrated medicated water" or "concentrate" means a dilution of the pharmaceutical aqueous suspension that is more concentrated than the medicated water and that can be used for further dilution at a place of use, e.g. a farm, for example by using a dosatron prior to consumption by animals.

In a first aspect, the invention relates to a pharmaceutical aqueous suspension comprising: at most 35 wt.% of flubendazole, based on the weight of the pharmaceutical aqueous suspension, as solid particles having a particle size distribution in which D50 ≤ 0.45 micrometer and ≥ 90 % of particles are ≤ 1.0 micrometer; a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25, one or more preservatives, and water.

The pharmaceutical suspension may in a specific embodiment comprise water, flubendazole, a co-solvent, a surfactant, one or more preservatives, an anti-foaming agent and a salt.

### Water

In an embodiment, water is present in an amount of between 20 and 70 wt.%, preferably between 40 and 60 wt.%, such as between 50 and 60 wt.% based on the weight of the pharmaceutical suspension. For the pharmaceutical aqueous suspension purified water is generally used. Any type of safe drinking water may be used for the (concentrated) medicated water. Depending on the national/regional pharmacopeia requirements, different levels of purified water may be required. Preferably, for the medicated water, water that is readily available at the site of use is used. The suspensions according to the invention have been tested in soft and hard waters. In the present description the following scales for hardness of water are used, these are according to EMEA/CVMP/540/03 Rev.1: Quality aspects of pharmaceutical veterinary medicines for administration via drinking water. Soft water/low pH is water having a pH range from 5.0 to 7.0 and 60 mg/L or less of calcium carbonate. Hard water/high pH is water having a pH range from 8.0 to 9.0 and between 180 to 350 mg/L of calcium carbonate.

### Active pharmaceutical ingredient (API)

*Flubendazole*

Flubendazole is the active pharmaceutical ingredient (API) in the present pharmaceutical aqueous suspension. Flubendazole [CAS 31430-15-6] is an anthelmintic, viz. an antiparasitic drug that can expel parasitic worms (helminths) - such as gastrointestinal roundworms (nematodes), respiratory roundworms (lungworms) and tapeworms - as well as other internal parasites from the body by either stunning or killing them and without causing significant damage to the host. The present invention is aimed at providing a novel pharmaceutical aqueous suspension of flubendazole that provides excellent stability in the suspension as such (for increased shelf life) as well as excellent stability in the medicated water that may be prepared using the pharmaceutical suspension.

The chemical structure of flubendazole is as follows:

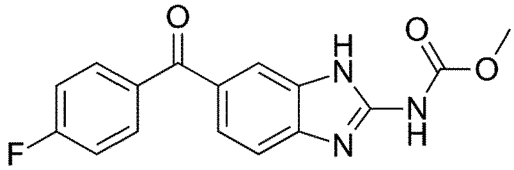

Flubendazole is present in solid form and forms the solid part of the suspension, whereas water (as suspending agent or solvent), optionally with a co-solvent and one or more other ingredients (e.g. preservative, surfactants, stabilizing agent, anti-foaming agent) form the liquid phase in which the solid particles of the API are suspended.

Flubendazole is present in an amount of at most 35 wt.%, based on the weight of the pharmaceutical aqueous suspension. Preferably, flubendazole is present in an amount of at least 10 wt.%, such as at least 15 wt.%. Relative higher concentrations of flubendazole in the pharmaceutical suspension are preferred to reduce transport cost (from the manufacturer to the farm). The present inventors have develop an optimal suspension that can have the highest concentration of flubendazole that still has good physical and chemical stability. In an embodiment, flubendazole is present in an amount of between 10 wt.% and 30 wt.%, such as between 15 wt.% and 25 wt.%, e.g. between 18 wt.% and 22 wt.% such as 20 wt.%. The present inventors have observed that when amounts higher (above 35 wt.%) than the claimed ranges are used, during the process to prepare the suspensions longer milling times where required. Tests with an API percentage of 40 wt.% were carried out and showed that the suspensions were very viscous and required very long milling times in manufacturing. The aim is to get a suspension with an API concentration that is as high as possible without providing issues with manufacturing and physical stability.

*Particle Size Distribution*

The present inventors have found that when the particle size distribution for flubendazole is in the range as claimed, flubendazole maintains in suspension both in the pharmaceutical product as in the medicated water for use. The present inventors have observed that the particle size distribution, especially the D50 and D90 values, are essential for obtaining good physical stability of the flubendazole in water. According to the present invention said flubendazole particles have a particle size distribution in which D50 ≤ 0.45 micrometer and D90 ≤ 1.0 micrometer (viz. ≥ 90 % of particles are ≤ 1.0 micrometer). According to the present invention, particle size distribution is measured using laser diffraction and hence it are volume percentages. A D50 of less than or equal to 0.45 micrometer means that 50 vol.% of all particles have a diameter that is 0.45 micrometer or less. More preferably, D50 ≤ 0.35 micrometer, even more preferably D50 ≤ 0.25 micrometer, most preferably D50 ≤ 0.20 micrometer. In an embodiment, D50 ≤ 0.18 micrometer or D50 ≤ 0.17 micrometer. A D90 of 1.0 micrometer means that 90 vol.% of all particles have a diameter that is 1.0 micrometer or less, or in other words ≥ 90 % of particles are ≤ 1.0 micrometer. More preferably at least 93.0 vol.% of all particles have a diameter that is 1.0 micrometer or less. The present inventors have observed that in order to obtain good stability of the suspension the particle size distribution of flubendazole in the suspension is essential.

**Surfactants**

The present inventors have observed that with the addition of a surfactant, the physical stability increases, in particular with poloxamer-type surfactants. A Poloxamer-type surfactant is required in the suspension according to the invention and is defined as a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25.

It is a specific aim of the present invention to get a combination of good (chemical and physical) stability on the one hand and optimal preservative efficacy. The skilled person is aware that when using a paraben-type preservatives in combination with surfactants this may lead to deactivation of the preservatives; e.g. Tween-type surfactants counter act the function of paraben-type preservatives.

The present inventors have now found that the combination of features of claim 1 provide the required stability. In particular, using a Poloxamer-type surfactant with a ratio of the PO and EO units as defined in the claims. The present inventors have found that the specific formulation using Poloxamer-type surfactant and the one or more preservative(s) balances the antimicrobial effect and the physical stability. In a preferred embodiment, the pharmaceutical aqueous suspension according to the present invention comprises a paraben-type preservative (or more than one) in combination with a poloxamer type surfactant.

Poloxamers are nonionic surfactants, being triblock copolymers having a central hydrophobic block, consisting of a poly(propylene oxide) (PPO or polyoxypropylene) flanked by two hydrophilic blocks, consisting of poly(ethylene oxide) (PEO or polyoxyethylene). Several types of poloxamers are commercially available having different lengths of the polymeric blocks each having a specific name starting with a P (for poloxamer) followed by three digits: the first two digits multiplied by 100 give the approximate molecular mass of the poly(propylene oxide)-block, and the last digit multiplied by 10 gives the percentage of poly(ethylene-oxide) content.

Preferably, the non-ionic triblock copolymer surfactant has a molecular weight of at least 9000 g/mole. Preferably, the non-ionic triblock copolymer surfactant is present in an amount of between 2 and 10 wt.% based on the weight of the pharmaceutical aqueous suspension. Preferably, the poly(propylene oxide)-block has a molecular mass of between 2000 and 6000, preferably between 3000 and 5000 gram/mole.

Poloxamer 407, which is a preferred surfactant according to the inventive, has 56 PO units, flanked by 100 EO units on either side. The ratio of PO units to EO units is 56 to 200, being 0.28. Poloxamer 407 has a poly(propylene oxide) molecular mass of 4000 g/mol. Preferably, a poloxamer-type surfactant having a molecular mass of the poly(propylene oxide) core of between 3000 and 5000 gram/mol is used. Poloxamer 188, which is not according to the present invention, has 27 PO units, flanked by 80 EO units on either side. The ratio of PO units to EO units is 27 to 160, being 0.169, being outside of the claimed scope. Poloxamer 188 has a poly(propylene oxide) molecular masses of 1800 gram/mol, which is not within the preferred range.

The present suspension preferably comprises the surfactant having in an amount of between 2 and 10 wt.%, preferably between 2 and 6 wt.%, or 3 and 5 wt.%, such as 4 wt.%. The present inventors have observed that this allows a good balance between solubility of the surfactant (which is less at higher values) and physical stability of the suspension (which is less at lower values).

Preservatives

The present inventors have observed that in order to obtain a suspension that is resistant against microbial growth, it is required to add a preservative. Since the present pharmaceutical aqueous suspensions may be used in the form of a so-called multidose suspensions in which aliquots are taken at the site of use from the container with the pharmaceutical aqueous suspension over time, a preservative is required. In other words, a container is used over time and after opening it is important to control microbiological quality. One or more preservatives are thus present in the present suspensions. Preferably, one or more preservatives of the so-called paraben-type are used; these are parahydroxybenzoates or esters of parahydroxybenzoic acid (also known as 4-hydroxybenzoic acid). More preferably, the present formulations comprises methyl para hydroxybenzoate (hereinafter methyl paraben), propyl para hydroxybenzoate (hereinafter propyl paraben) or even more preferably a combination thereof. A combination of methyl paraben and propyl paraben provide a good balance between the high potency of the propyl paraben and the higher solubility of the methyl paraben. In an embodiment, two preservatives of the paraben-type are present being methyl paraben and propyl paraben, preferably in a ratio of between 10:1 and 1:1, such as between 5:1 and 3:1, e.g. 3.6:1. In an embodiment, the one or more preservatives are present in a total amount of between 0.1 and 0.6 wt.%, preferably between 0.2 and 0.5 wt.% based on the weight of the pharmaceutical aqueous suspension.

**Co-solvent/co-suspending agents**

One or more co-solvents (co-suspending agents) may be present. For example, a co-solvent may be added for additional preservative action and/or in order to improve the solubility of the one or more preservatives in the suspension. In an embodiment, the suspensions further comprises an alkylene glycol as a water-soluble co-solvent, preferably wherein said alkylene glycol is polyethylene glycol (PEG) or propylene glycol (PG) most preferably propylene glycol. The present inventors have observed that when using an alkylene glycol water-soluble co-solvent good results are obtained in combination with the use of one or more paraben-type preservatives. More specifically, the present inventors have observed that good results can be obtained with the use of propylene glycol combined with the paraben-type preservatives discussed above. The amount of co-solvent may be between 0 and 30 wt.% based on the weight of the pharmaceutical aqueous suspension, such as between 15 and 25 wt.%, more preferably between 17 and 21 wt.%, for example 20 wt.%.

**Other additives**

One or more other additives may be present in the pharmaceutical aqueous suspensions according to the present invention. Sodium chloride may be present as an additive. Preferably, sodium chloride is used an amount of between 0.1 and 1.0 wt.%, preferably between 0.25 and 0.6 wt.%, such as between 0.35 and 0.5 wt.%. The present inventors have observed that the presence of sodium chloride has a beneficial effect on the physical stability and preservative effectiveness. One or more antifoaming agents may be present in the pharmaceutical aqueous suspensions

according to the present invention. Simethicone (e.g. as an emulsion) may be present as an antifoaming agent. In an embodiment, the suspension comprises simethicone as antifoaming agent in an amount of between 0.1 and 1.0 wt.%, such as between 0.2 and 0.8 wt.%, or 0.3 and 0.7 wt.%, preferably between 0.4 and 0.6 wt.%, such as 0.5 wt.% based on the weight of the pharmaceutical aqueous suspension. The present inventors have observed that with amounts of between 0.1 and 0.3 wt.% limited antifoaming activity was observed and that with amounts between 0.4 and 0.6 wt.% optimal antifoaming activity was observed. Higher amounts may be used but do not provide additional effect.

**Specific embodiments of the pharmaceutical aqueous suspension**

In an embodiment, the invention relates to a pharmaceutical aqueous suspension being an aqueous suspension of:

* solid flubendazole particles in an amount of between 15 and 25 wt.% of flubendazole, based on the weight of the pharmaceutical aqueous suspension, said flubendazole particles having a particle size distribution in which D50 ≤ 0.45 micrometer and ≥ 90 % of particles are ≤ 1.0 micrometer;
* a non-ionic triblock copolymer surfactant comprising a central poly(propylene oxide)-block having at least 30 propylene oxide (PO) units and having a poly(propylene oxide)-block molecular mass between 3000 and 5000 gram/mole, said central poly(propylene oxide)-block being flanked by two poly(ethylene oxide)-blocks, each having at a certain number of ethylene oxide (EO) units, wherein the ratio of number of PO units to total number of EO units is at least 0.25;
* one or more preservatives of the paraben-type;
* an alkylene glycol;
* water; and
* said pharmaceutical aqueous suspension being physically and chemically stable at a temperature of 25 °C.

In an embodiment, the invention relates to a pharmaceutical aqueous suspension being an aqueous suspension of:

* solid flubendazole particles in an amount of between 15 and 25 wt.% of flubendazole, based on the weight of the pharmaceutical aqueous suspension, said flubendazole particles having a particle size distribution in which D50 ≤ 0.45 micrometer and ≥ 90 % of particles are ≤ 1.0 micrometer;
* a non-ionic triblock copolymer surfactant comprising a central poly(propylene oxide)-block having at least 30 propylene oxide (PO) units and having a poly(propylene oxide)-block molecular mass between 3000 and 5000 gram/mole, said central poly(propylene oxide)-block being flanked by two poly(ethylene oxide)-blocks, each having at a certain number of ethylene oxide (EO) units, wherein the ratio of number of PO units to total number of EO units is at least 0.25;
* methyl paraben and propyl paraben as preservatives of the paraben-type;
* sodium chloride;
* an alkylene glycol;
* water; and
* said pharmaceutical aqueous suspension being physically and chemically stable at a temperature of 25 °C.

In an embodiment, the invention relates to a pharmaceutical aqueous suspension being an aqueous suspension of:

* solid flubendazole particles in an amount of between 15 and 25 wt.% of flubendazole, based on the weight of the pharmaceutical aqueous suspension, said flubendazole particles having a particle size distribution in which D50 ≤ 0.45 micrometer and ≥ 90 % of particles are ≤ 1.0 micrometer;
* a non-ionic triblock copolymer surfactant comprising a central poly(propylene oxide)-block having at least 30 propylene oxide (PO) units and having a poly(propylene oxide)-block molecular mass between 3000 and 5000 gram/mole, said central poly(propylene oxide)-block being flanked by two poly(ethylene oxide)-blocks, each having at a certain number of ethylene oxide (EO) units, wherein the ratio of number of PO units to total number of EO units is at least 0.25; in between 2 and 6 wt.% based on the weight of the pharmaceutical aqueous suspension;
* one or more preservatives of the paraben-type in a total amount of between 0.1 and 1.0 wt.% based on the weight of the pharmaceutical aqueous suspension;
* sodium chloride in an amount of between 0.1 and 1.0 wt.% based on the weight of the pharmaceutical aqueous suspension;
* an alkylene glycol as a water-soluble co-solvent in an amount of between 15 and 25 wt.% based on the weight of the

pharmaceutical aqueous suspension;
* water in an amount of between 20 and 70 wt.% based on the weight of the pharmaceutical aqueous suspension; and
* said pharmaceutical aqueous suspension being physically and chemically stable at a temperature of 25 °C.

In a very specific embodiment, the invention relates to a pharmaceutical aqueous suspension being an aqueous suspension of:

* solid flubendazole particles in an amount of between 15 and 25 wt.% of flubendazole, based on the weight of the pharmaceutical aqueous suspension, said flubendazole particles having a particle size distribution in which D50 ≤ 0.45 micrometer and ≥ 90 % of particles are ≤ 1.0 micrometer;
* a non-ionic triblock copolymer surfactant comprising a central poly(propylene oxide)-block having at least 30 propylene oxide (PO) units and having a poly(propylene oxide)-block molecular mass between 3000 and 5000 gram/mole, said central poly(propylene oxide)-block being flanked by two poly(ethylene oxide)-blocks, each having at a certain number of ethylene oxide (EO) units, wherein the ratio of number of PO units to total number of EO units is at least 0.25; in an amount of between 2 and 6 wt.% based on the weight of the pharmaceutical aqueous suspension;
* methyl paraben and propyl paraben as preservatives in a total amount of between 0.1 and 1.0 wt.% based on the weight of the pharmaceutical aqueous suspension;
* sodium chloride in an amount of between 0.1 and 1.0 wt.% based on the weight of the pharmaceutical aqueous suspension;
* a propylene glycol as a water-soluble co-solvent in an amount of between 15 and 25 wt.% based on the weight of the pharmaceutical aqueous suspension;
* water in an amount of between 40 and 60 wt.% based on the weight of the pharmaceutical aqueous suspension; and
* said pharmaceutical aqueous suspension being physically and chemically stable at a temperature of 25°C.

**(Concentrated) medicated water**

The present invention also relates to a (concentrated) medicated water comprising water and the pharmaceutical aqueous suspension in an amount of between 0.1 ml and 150 ml per liter of water. When using this range and when using a API concentration of 20 wt.% in the pharmaceutical aqueous suspension, the amount of flubendazole in the medicated water ranges between 0.02 g and 30 gram per liter, being between 0.002 and 3 wt.% of flubendazole. The container comprising the pharmaceutical aqueous suspension should be shaken for 30 seconds before use. When a concentrated medicated water is used, this should be further diluted prior to use according to methods known in the field.

**Stability of pharmaceutical aqueous suspension and (concentrated) medicated water**

The aim of the present invention is to obtain a pharmaceutical aqueous suspension with flubendazole as well as a (concentrated) medicated water prepared therewith, all having excellent physical and chemical stability. The main aim of the present invention is to provide a physically stable flubendazole pharmaceutical aqueous suspension as well as a physically stable (concentrated) medicated water.

*Physical stability*

The physical stability of the pharmaceutical aqueous suspension was tested by testing two types of tests: i) the particle size distribution after storing (stability against aggregation); and ii) the re-suspensibility after storing (re-suspensible).

The physical stability for all of the above cited tests is tested after storing at room temperature (according to the present invention, room temperature is set at 25 °C) since it is desirable for storing purposes that the suspension does not have to be kept refrigerated. In addition, the physical stability for the pharmaceutical aqueous suspension is tested after storing at a temperature of 30 °C.

For the pharmaceutical aqueous suspension a long shelf life of at least 6 months (6M), preferably at least 12 months (12M), more preferably at least 18 months (18M), even more preferably at least 24 months (24M) or even at least 36 months (36M) is desirable for storing purposes. Any sediment that may have formed during the storing of the pharmaceutical aqueous suspension should be able to be resuspended within 30 seconds. Moreover, during this time of storing the particle size distribution should remain within the claimed scope. The pharmaceutical aqueous suspension is considered to be stable if after the above cited storing (6M to 36M) it can be resuspended within 30 seconds and if the particle size distribution is within the claimed range. Therefore, the two above tests (stability against aggregation and re-suspensibility) are used to determine the physical stability of the pharmaceutical aqueous suspension.

For the (concentrated) medicated water (diluted pharmaceutical aqueous suspension) the physical stability

needs to be 24 hours, since it is prepared and directly provided to the animals for drinking. For dosing to the animals it is important that the medicated water is homogenous. With homogeneous in the present context is meant that the concentration of particles in the same throughout the medicated water (no sedimentation) and that the particle size is within the claimed range (no aggregation). A shelf life of 24 hours was considered sufficient for (concentrated) medicated water, during which time it remained homogeneous (no aggregation and no sedimentation). Therefore, the two above tests (stability against aggregation and stability against sedimentation) are used to determine the physical stability of the (concentrated) medicated water.

*Pharmaceutical aqueous suspension*

As cited above, the physical stability of the pharmaceutical aqueous suspension was tested by i) the particle size distribution after storing; and ii) the re-suspensibility after storing.

iii) the particle size distribution after storing

A first test for the physical stability of the pharmaceutical aqueous suspension is if the pharmaceutical aqueous suspension complies with the claimed particle size distribution after storing. This test shows if any aggregation has occurred during storing. The pharmaceutical aqueous suspension is tested after storing for the cited number of months (6M - 36 M). After storing first the suspension is manually shaken to ensure a homogeneous suspension (manual shaking for 30 seconds according to the method disclosed for measuring the resuspendability) and then a sample is taken 1 cm from top of the suspension.

In the context of the present invention, the pharmaceutical aqueous suspension is considered to be stable against aggregation if after storing the pharmaceutical aqueous suspension for a certain number of months (6, 12, 18, 24 or 36 months) at a certain temperature (either 25 ° ± 2°C or 30° ± 2°C), the flubendazole has a particle size distribution in which D50 ≤ 0.45 micrometer and ≥ 90 % of particles are ≤ 1.0 micrometer. The pharmaceutical aqueous suspension preferably has a stability against aggregation of at least 6 months, preferably at least 12 months, more preferably at least 18 months, even more preferably at least 24 months, most preferably at least 36 months at 25 °C. The pharmaceutical aqueous suspension preferably has a stability against aggregation of at least 6 months, preferably at least 12 months, more preferably at least 18 months, even more preferably at least 24 months, most preferably at least 36 months at 30 °C. The pharmaceutical aqueous suspension most preferably has a stability against aggregation of at least 36 months at 30°C.

ii) the resuspendability after storing

Another test for the physical stability of the pharmaceutical aqueous suspension is the determination of the resuspendability after storing. The sample is shaken (see experimental section for details) for 30 seconds and visually observed if after the shaking there is no remaining caking (visual homogenous composition, free from sediment).

In the context of the present invention, the pharmaceutical aqueous suspension is considered to be resuspensible if after storing the pharmaceutical aqueous suspension for a certain number of months (6, 12, 18, 24 or 36 months) at a certain temperature (either 25 ° ± 2°C or 30° ± 2°C), can be resuspended in 30 seconds. Preferably, the pharmaceutical aqueous suspension can be resuspended after storing for at least 36 months at 25 °C in 30 seconds.

*(Concentrated) medicated water*

As cited above the physical stability of the (concentrated) medicated water was tested by i) the particle size distribution after storing; and iii) the amount of sedimentation after storing.

iii) the particle size distribution after storing

A first test for the physical stability is if the (concentrated) medicated water complies with the claimed particle size distribution after storing for 24 hours. After storing first the suspension is manually shaken to ensure a homogeneous suspension (manual shaking for 30 seconds according to the method disclosed for measuring the resuspendability) and then a sample is taken 1 cm from top of the suspension. This test shows of any aggregation has occurred during storing. In the context of the present invention, the (concentrated) medicated water is considered to be stable against aggregation if after storing the (concentrated) medicated water for 24 hours at 25 ° ± 2°C, the flubendazole has a particle size distribution in which D50 ≤ 0.45 micrometer and ≥ 90 % of particles are ≤ 1.0 micrometer.

iii) the amount of sedimentation after storing

Sedimentation was tested by determining the amount of flubendazole after a certain period of storing time from samples taken at or near the bottom of the storing container and near the top of the storing container. The difference between the values near the top and the bottom of the container provide information regarding the amount of sedimentation; if the amount at or near the bottom is significantly higher than the amount near the top, sedimentation has occurred.

In the context of the present invention, the (concentrated) medicated water is considered to be stable against sedimentation if after storing the (concentrated) medicated water for 24 hours at 25 ° ± 2°C, the amount of flubendazole near the top as well as the amount of flubendazole at or near the bottom are both between 90% and 110% of the calculated (theoretical) amount of flubendazole. The (concentrated) medicated water preferably has a stability against sedimentation of at 24 hours at 25 °C.

With "calculated (theoretical) amount of flubendazole" in this context is meant the amount of flubendazole that is calculated to be present based on the dilution that is used to prepare the (concentrated) medicated water.

When the stability is tested of a medicated water in with a flubendazole composition comprising 200 mg/ml of flubendazole is diluted (1 ml per liter water) to a final concentration of flubendazole of 0.2 mg/ml, the medicated water is considered stable if the amount tested near the top after 24 hours at 25 °C (± 2°C) and 60 %RH is between 0.18 and 0.22 mg/ml and if the amount tested at or near the bottom after 24 hours at 25 °C (± 2°C) and 60 %RH is between 0.18 and 0.22 mg/ml. If after this testing both samples (top and bottom) are within the range of 90% to 110% of said calculated flubendazole amount, the medicated water is considered to be stable against sedimentation. When the stability is tested of a concentrated medicated water in with a flubendazole composition comprising 200 mg/ml of flubendazole is diluted (100 ml per liter water) to a final concentration of flubendazole of 20 mg/ml, the concentrated medicated water is considered stable if the amount tested near the top after 24 hours at 25 °C (± 2°C) and 60 %RH is between 18 and 22 mg/ml and if the amount tested at or near the bottom after 24 hours at 25 °C (± 2°C) and 60 %RH is between 18 and 22 mg/ml. If after this testing both samples (top and bottom) are within the range of 90% to 110% of said calculated flubendazole amount, the concentrated medicated water is considered to be stable against sedimentation. In other words, the amount of flubendazole after storing both near the top and at or near the bottom is within a range of ±10% compared to the flubendazole amount measured prior to storing.

*Chemical stability*

The chemical stability was tested by reviewing the levels of flubendazole and degradation products thereof (also called impurities) after a certain period of storing time (shelf life). This shows if some of the flubendazole has degraded to other compounds. Chemical degradation of flubendazole in (concentrated) medicated water is assessed by a decrease in the levels of flubendazole (if any) in the medicated water, viz. by an assay of the amount of flubendazole and by determining the levels of impurities (total impurities).

In the context of the present invention, the pharmaceutical aqueous suspension is considered to be chemically stable if after storing the pharmaceutical aqueous suspension for a certain number of months (6, 12, 18, 24 or 36 months) at a certain temperature (either 25 ° ± 2°C or 30° ± 2°C), the amount of flubendazole is between 95% and 105% of the theoretical amount of flubendazole.

With "theoretical amount of flubendazole" in this context is meant the amount of flubendazole that is be present in the composition, in other words that should be present based on the label. When the chemical stability is tested of a flubendazole composition comprising 200 mg/ml of flubendazole, it is considered stable if the amount tested after the cited number of months at the cited temperature (± 2°C) is between 190 and 210 mg/ml. When a pharmaceutical aqueous suspension is marketed it has a certain amount of active ingredient mentioned on the label, if after this testing the sample is within the range of 95% to 105% of said labeled amount, the pharmaceutical aqueous suspension is considered to be chemically stable. In other words, the amount of flubendazole after storing is within a range of ±5% compared to the theoretical amount of flubendazole.

The pharmaceutical aqueous suspension preferably has a chemical stability of at least 6 months, preferably at least 12 months, more preferably at least 18 months, even more preferably at least 24 months, most preferably at least 36 months at 25 °C. The pharmaceutical aqueous suspension preferably has a chemical stability of at least 6 months, preferably at least 12 months, more preferably at least 18 months, even more preferably at least 24 months, most preferably at least 36 months at 30 °C. The pharmaceutical aqueous suspension most preferably has a chemical stability of at least 36 months at 30 °C.

Medical **Uses of the pharmaceutical compositions**

The invention relates to a pharmaceutical aqueous suspension for use in the treatment of internal parasites in mammals, such as poultry and pigs. The present invention also relates to the use of a pharmaceutical aqueous suspension

for the preparation of a medicated water to provide a dosage of between 1 and 2.5 mg of flubendazole per kg of body weight per day. In a specific use, the dosage for chickens may be 1.43 mg of flubendazole per kg of body weight daily for 7 consecutive days. In particular for the treatment of helminthiasis caused by *Ascaridia galli* (adult stages), *Heterakis gallinarum* (adult stages), and/or *Capillaria spp.* (adult stages). In a specific use, the dosage for pigs for treatment of helminthiasis caused by adult stages and L4 intestinal stages of *Ascaris suum* may be 1 mg of flubendazole per kg of body weight daily for 5 consecutive days. In a specific use, the dosage for pigs for treatment of adult stages of *Ascaris suum* may be 2.5 mg of flubendazole per kg of body weight daily for 2 consecutive days.

**Process of** preparing the pharmaceutical aqueous suspension

The present invention relates to process of preparing the above pharmaceutical aqueous suspension comprising the steps of:

* preparing a mixture of solid flubendazole particles, a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25, and water;
* milling said mixture so obtain an aqueous suspension; and
* adding one or more preservatives to said aqueous suspension;

in order to obtain a pharmaceutical aqueous suspension composition comprising at most 35 wt.% of flubendazole, based on the weight of the pharmaceutical aqueous suspension, as solid particles having a particle size distribution in which D50 $\leq$ 0.45 micrometer and $\geq$ 90 % of particles are $\leq$ 1.0 micrometer.

In an embodiment, an antifoaming agent may be added in the first step of preparing a mixture. The milling may be carried out using a mill that is known to a person skilled in the art, such as a high-efficiency agitator bead mill. Milling is carried out to decrease the size of the flubendazole particles to solid particles having a particle size distribution in which D50 $\leq$ 0.45 micrometer and $\geq$ 90 % of particles are $\leq$ 1.0 micrometer. After the milling the one or more preservatives are added to the mixture. The one or more preservatives may be added as a preservative solution in a co-solvent. Thereafter, an optional step of adding a salt may be carried out, preferably sodium chloride, more preferably an aqueous solution of sodium chloride.

The process according to the present invention leads to the pharmaceutical aqueous suspension according to the invention.

Effects of the invention

With the pharmaceutical aqueous suspension according to the present invention, this stability issues of may prior art flubendazole suspensions is improved as it has been found to show excellent physical stability, chemical stability, or both.

The present pharmaceutical aqueous suspension is very suitable for administration via the drinking water systems that are already present in animal keeping and/or production facilities without need for (significant) modifications.

The pharmaceutical aqueous suspension having the flubendazole particle size complying to the claimed specification and with the poloxamer type surfactant show hardly any sedimentation or segregation in medicated drinking water. Homogeneity of (concentrated) medicated drinking water has been proven by determining the assay and PSD of flubendazole at top and bottom of vessels at initial and after 24 hours of storage. This proves that accurate dosing is guaranteed with the pharmaceutical aqueous suspension according to the present invention.

Hence one or more aims of the present invention as solved by the claimed invention.

EXAMPLES

The present invention is further elucidated based on the Examples below which are illustrative only and not considered limiting to the present invention.

<u>Pharmaceutical aqueous suspensions</u>

Materials

Flubendazole was obtained from Changzhou Yabang-QH Pharmachem Co. Ltd.
Methyl parahydroxyybenzoate and propyl parahydroxybenzoate were obtained from Salicylates and Chemicals Pvt. Ltd.
Propylene glycol was obtained from Dow Chemical Com./Ravago
Poloxamer 407 was obtained from BASF
Sodium chloride was obtained from Kemika d.d.
Simethicone emulsion was obtained from Dow Corning GmBH
Water, purified was prepared (Ph.Eur 0008) at the Dechra manufacturing site

Method for preparation

A pharmaceutical aqueous suspension according to the present invention was prepared according to the specifications shown below in Table 1.

Table 1. ingredients of pharmaceutical aqueous suspension.

| **Ingredient** | **Amount per ml** | **wt.%** | **Function** |
|---|---|---|---|
| Flubendazole | 200.0 mg | 20.0 wt.% | API |
| Methyl paraben | 2.7 mg | 0.27 wt.% | Preservative |
| Propyl paraben | 0.75 mg | 0.075 wt.% | Preservative |
| Propylene glycol | 200.0 mg | 20.0 wt.% | Co-solvent |
| Poloxamer 407 | 40.0 mg | 4.0 wt.% | Surfactant |
| Sodium chloride | 3.5 mg | 0.35 wt.% | Additive |
| Simethicone emulsion | 5.0 mg | 0.50 wt.% | Antifoam agent |
| Water, purified | Up to 1.0 ml | 54.8 wt.% | Suspending agent/solvent |

For the preparation of a 390 liter batch, first, the surfactant (15.6 kg), simethicone emulsion (1.95 kg), and purified water (153.27 kg) were mixed in a compounding tank until a homogeneous emulsion was obtained. Then flubendazole (78.00 kg) was added and stirred until a homogeneous suspension was obtained. Thereafter, the mixture obtained was milled using a high-efficiency agitator bead mill for a duration sufficient to achieve the desired particle size distribution. An aqueous suspension was obtained wherein the size of the flubendazole particles to solid particles having a particle size distribution in which D50 ≤ 0.45 micrometer and 96 % ≤ 1.0 micrometer. Then, a solution of methyl paraben and propyl paraben in propylene glycol was prepared and added to compounding tank and mixed. Thereafter, an aqueous solution of 1.365 kg of sodium chloride and 93.347 kg of purified water was added to the aqueous suspension and stirred until a homogeneous suspension was obtained.

**Methods for measurements**

*Method for determining the amount of flubendazole in the pharmaceutical aqueous suspension*

The identification of the active substance flubendazole in the pharmaceutical aqueous suspension consists of two complementary tests by means of a combined HPLC/UV-VIS diode array method. The first test compares the retention time of the sample solution with the retention time of the reference solution obtained by the HPLC method. For the second test the diode array spectrum of the flubendazole peak of the sample solution is compared with the one of the reference solution.

The reference solution for determining the amount of flubendazole in the pharmaceutical aqueous suspension is 200 mg of flubendazole in a 100 ml volumetric flask dissolved in approximately 50 ml formic acid and diluted to 100 ml with solvent (70:30 ddH-$_2$O:acetonitrile). The sample solution is obtained by weighing 542 mg of the pharmaceutical aqueous suspension to be tested in a 50 mL volumetric flask. The solution is further dissolved in 25 mL formic acid and immediately diluted to 50 mL with solvent (70:30 ddH-$_2$O:acetonitrile). Reference and sample solutions are stable for up to 7 days when stored at ambient conditions in clear colorless volumetric flask, at 20 °C in an amber HPLC vial or at 2 - 8 °C in an amber HPLC vial.

A Waters Acquity C18 BEH, 50 x 3.0 mm; 1.7 micrometer column is used, using a reversed phase gradient system with a mobile phase A and a mobile phase B ad a 0.8 ml per minute flow rate and a total run time of 13.0 minutes. Mobile phase A being prepared by dissolving 848 mg of ammonium acetate in 900 ml of purified water and adjusting the pH with

2.5M sodium hydroxide until pH 9.85 (±0.04) and then adding 100 ml acetonitrile and homogenize. Pure acetonitrile is used as mobile phase B. The gradient is as follows: 0 minutes 100 % A (0% B); 10 minutes 35 % A and 65 % B; 10.1 minutes 100% B (0% A); 13 minutes 100% B (0% A). The column temperature is 40 °C and the autosampler temperature is 20 °C. The injection volume is 5 microliter. The UV/VIS channel is set to 249 nm for flubendazole. Data is collected with a rate of 10 Hz with a response time of 1 second. Moreover, for the Diode array test a 3D field with wavelengths between 210 nm and 600 nm was tested with a bunch width of 2 nm (distance between recorded data points in a 3D field). The retention time for the peak of flubendazole (5.41 minutes) obtained with the sample solution is the same as the reference solution. The diode array graphs obtained with the sample solution have similar shape as graphs obtained with the reference solution.

The concentration of flubendazole in the sample of the pharmaceutical aqueous suspension is calculated by the following formula:

$$Flubendazole\ (mg/mL) = \frac{[\mathrm{Reference}_{mg/ml}] * Area\ Sample}{[\mathrm{Sample}_{g/ml}] * Area\ Reference} * Purity\ Standard * Density\ of\ sample_{(at\ 20°C)}$$

The area of the sample and the area of the reference are the area under the curve and are directly exported from the software used. The purity factor is taken from the certificate of analysis of the standard and can vary slightly from lot to lot of the standard used. The density in the relative density and is determined using a pycnometer at 20 °C.

*Method for determining the amount of (impurities/degradation products):*

There are a series of known degradation products from flubendazole of which the amounts are tested in very similar way as shown above for measuring the amount of flubendazole using HPLC. These known degradation products are methyl[5-[4-(formylamino)benzoyl]-1H-benzimidazol-2-yl]carbamate (retention time 3.36 minutes, response factor 0.69), 2-amino-1H-benzoimidazol-5-yl)(4-fluorophenyl)methanone (retention time 4.37 minutes, response factor 0.96), 4-fluoro-phenyl(2-hydroxy-1H-benzimidazol-5-yl)methanone (retention time 4.51 minutes, response factor 0.84), 1H-benazimidazol-5-yl(4-fluorophenyl)-methanone (retention time 4.67 minutes, response factor 0.83), methyl [5-(2-fluorobenzoyl)-1H-benzimidazol-2-yl]carbamate (retention time 5.09 minutes, response factor 0.98), methyl[5-(4-fluorobenzoyl)-1-methyl-1H-benzimidazol-2-yl]carbamate (retention time 5.76 minutes, response factor 1.07), and methyl[5-[4-(1-methoxylethoxy)benzoyl]-1H-benzimidazol-2-yl]carbamate (retention time 6.61 minutes, response factor 0.75).

The amount of all known degradation products is calculated using the following equation:

$$\text{Known related substances (\%)} = \frac{Area_{known}/RF_{known}}{Area_{substance} + Total\ area_{unknown} + \sum_{i=1}^{7}\left(\frac{Area_{known\ i}}{RF_{known\ i}}\right)} * 100\%$$

Unknown related substances are calculated similar to the method for determining the amount of flubendazole. The concentration of each individual unknown related substances is calculated by the formula:

$$\text{Unknown related substances (\%)} = \frac{Area_{unknown}}{Area_{substance} + Total\ area_{unknown} + \sum_{i=1}^{7}\left(\frac{Area_{known\ i}}{RF_{known\ i}}\right)} * 100\%$$

Wherein for both of the above equations: $area_{known}$ is the area under the curve of the peaks of all known substances, total $area_{unknown}$ is the area under the curve of the peaks of all unknown substances, $area_{substance}$ is the area under the curve of the flubendazole peak and $area_{known\ i}$ is the specific area under the curve for one specific known compound and $RF_{known\ i}$ is the response factor for one specific known compound.

*Method for determining the particle size distribution of flubendazole:*

Particle size of flubendazole in the pharmaceutical aqueous suspension according to the invention was measured by first manual shaking the containers for 30 seconds (see method for resuspension) and then 0.5 ml of the suspension was taken and diluted with 50 ml degassed purified water and stirred to obtain a homogeneous suspension. A Malvern Mastersizer 2000 is used using a Hydro 2000 SM sample handling unit, with a measuring time of 10 seconds and obscuration limits of 6-7 %. The stirring speed of the pump is set to 2000 rpm. The measurement should be started within 3 minutes.

*Method for determining the resuspension*

The testing is performed by manual shaking for 30 seconds, using a calibrated timer. After 30 seconds, a visual assessment is performed for potential sediment and a quantitative assay of flubendazole is performed for the homogeneity of the suspension. The procedure for manual shaking of the 250 ml container is as follows: with the container in one hand, hold the container inverted. Shake the container with vertical movements for total 30 seconds. During the 30 seconds period, quickly turn the container in upright position and back inverted again at t=5, 10, 15, 20 and 25 seconds. The sedimentation is visually checked by placing the container in a position so that the liquid level is on the container of the container and observe if the container is free of sediment. If needed, a light source from above the container can be used to improve the visual assessment. For example a flashlight can be used as a light source.

**Physical stability for the pharmaceutical aqueous suspension**

In the tables below the resuspendability is provided as well as the stability against aggregation, both measures of the physical stability. Firstly, the concentration of flubendazole in the pharmaceutical aqueous suspension was analyzed at the top and bottom of a container.

Four samples in 250 ml containers were stored for 36 months each under the following conditions: i) stored in upright position at 25 °C (± 2 °C) and 60 % RH (±5%); ii) stored in inverted position at 25 °C (± 2 °C) and 60 % RH (±5%); iii) stored in upright position at 30 °C (± 2 °C) and 65 % RH (±5%); and iv) stored in inverted position at 30 °C (± 2 °C) and 65 % RH (±5%). All samples were measured at 0, 3, 6, 9, 12, 18, 24, 30 and 36 months (denoted in the tables below as 0M, 3M, 6M, 9M, 12M, 18M, 24M, 30M, and 36M respectively). The pharmaceutical aqueous suspensions were stored in a container of 250 mL of HPDE with a LDPE seal in contact with the suspension during the stability tests. A significant variation in the values at the top and bottom could indicate instability of the pharmaceutical aqueous suspension due to sedimentation of the solid flubendazole particles from the suspension. The visual appearance at the start (t=0 months) for all samples, has to be a white to off-white suspension. Subsequently, for all samples, every measuring point had a similar visual appearance compared to the start.

"Particle size" as referred to hereafter is the median particle diameter (also referred to as the D50) of the particle size distribution. For acceptable results of the pharmaceutical aqueous suspension according to the invention, the D50 particle size should be less than 0.45 micron (Table 2), and > 90 % of particles should be less than 1.0 micron (Table 3). The four samples as indicated above are determined over time (t) as measured in months, values not determined (n.d.) are indicated as such.

Table 2. Stability against aggregation for pharmaceutical aqueous suspension; D50 value, median particle size in micron.

| Sample | 0M | 3M | 6M | 9M | 12M | 18M | 24M | 30M | 36M |
|---|---|---|---|---|---|---|---|---|---|
| **25°C/60%RH upright** | 0.16 | 0.16 | 0.16 | 0.16 | 0.16 | 0.16 | 0.15 | 0.15 | 0.16 |
| **25°C/60%RH inverted** | 0.16 | n.d. | n.d. | n.d. | 0.16 | n.d. | 0.15 | n.d. | 0.16 |
| **30°C/65%RH upright** | 0.16 | n.d. | n.d. | n.d. | 0.16 | 0.16 | 0.16 | n.d. | 0.17 |
| **30°C/65%RH inverted** | 0.16 | n.d. | n.d. | n.d. | 0.16 | n.d. | 0.16 | n.d. | 0.17 |

Table 3. Stability against aggregation for pharmaceutical aqueous suspension, percentage of particles less than 1.0 micron

| sample | 0M | 3M | 6M | 9M | 12M | 18M | 24M | 30M | 36M |
|---|---|---|---|---|---|---|---|---|---|
| **25°C/60%RH upright** | 96% | 95% | 95% | 95% | 95% | 95% | 95% | 95% | 95% |
| **25°C/60%RH inverted** | 96% | n.d. | n.d. | n.d. | 96% | n.d. | 96% | n.d. | 96% |
| **30°C/65%RH upright** | 96% | n.d. | n.d. | n.d. | 95% | 95% | 95% | n.d. | 94% |
| **30°C/65%RH inverted** | 96% | n.d. | n.d. | n.d. | 95% | n.d. | 95% | n.d. | 94% |

From the above data in Tables 2 and 3 it can be observed that for all four samples, the values are within the preferred range. In addition, the data clearly shows that the suspensions are completely physically stable against aggregation over a period of 36 months at 25 °C or even 30 °C. Results further show no agglomeration or aggregation as particle size distribution values remain stable over time.

To analyze the physical stability of the pharmaceutical aqueous suspension according to the invention the

resuspendability was analyzed. The term "resuspendability" as referred herein indicates if the suspension is fully resuspended after 30 seconds of manual shaking.

Table 4. Resuspendability for pharmaceutical aqueous suspension.

| sample | 0M | 3M | 6M | 9M | 12M | 18M | 24M | 30M | 36M |
|---|---|---|---|---|---|---|---|---|---|
| 25°C/60%RH upright | yes | yes | yes | yes | yes | yes | yes | yes | yes |
| 25°C/60%RH inverted | yes | n.d. | n.d. | n.d. | yes | n.d. | yes | n.d. | yes |
| 30°C/65%RH upright | yes | n.d. | n.d. | n.d. | yes | yes | yes | n.d. | yes |
| 30°C/65%RH inverted | yes | n.d. | n.d. | n.d. | yes | n.d. | yes | n.d. | yes |

From the above data in Table 4 it can be observed that for all samples the suspensions are resuspendible. Results show that resuspendability does not alter over a period of 36 months at 25 °C or 30 °C.

Together, the two above analyses on the physical stability of the pharmaceutical aqueous suspension according to the invention is stable and does not form agglomerates or aggregates and is readily resuspendible over a period of up to (and presumably longer than) 36 months.

**Chemical stability for the pharmaceutical aqueous suspension**

After having tested the physical stability of the pharmaceutical aqueous suspension according to the invention, the chemical stability of flubendazole in the suspension was tested. To this end, the four samples as described above were analyzed for the concentration (mg/L) of flubendazole before (0M) and after storage for up to 36 months (36M). For all samples, the amount of flubendazole should be between 190 and 210 milligram per liter (theoretical amount being 200 milligram/liter) to be considered chemically stable. In addition, the amount of impurities derived from chemical degradation of flubendazole, being a measure of the chemical stability are provided. The amount of impurities should be less than 2 %.

Table 5. Concentration of flubendazole before and after storage of pharmaceutical aqueous suspension. Concentration in mg/L.

| sample | 0M | 3M | 6M | 9M | 12M | 18M | 24M | 30M | 36M |
|---|---|---|---|---|---|---|---|---|---|
| 25°C/60%RH upright | 197 | 203 | 200 | 201 | 202 | 201 | 202 | 201 | 202 |
| 25°C/60%RH inverted | 197 | n.d. | n.d. | n.d. | 202 | n.d. | 201 | n.d. | 202 |
| 30°C/65%RH upright | 197 | n.d. | n.d. | n.d. | 202 | n.d. | 201 | n.d. | 201 |
| 30°C/65%RH inverted | 197 | n.d. | n.d. | n.d. | 203 | n.d. | 202 | n.d. | 201 |

From the above data it is observed that for all four samples, the values are within the preferred range. This indicates that the suspensions are completely chemically stable over a period of 36 months at 25 °C or 30 °C.

Next, the amount of impurities and/or degradation products that form within the suspension over a period of up to 36 months was analyzed. Preferred is that the amount is less than or equal to 2 %.

Table 6. Amount of degradation products in pharmaceutical aqueous suspension. Amounts in percentages per weight (wt.%).

| sample | 0M | 3M | 6M | 9M | 12M | 18M | 24M | 30M | 36M |
|---|---|---|---|---|---|---|---|---|---|
| 25°C/60%RH upright | 1.1 | 0.9 | 1.1 | 1.0 | 0.9 | 0.9 | 1.0 | 0.7 | 0.9 |
| 25°C/60%RH inverted | 1.1 | n.d. | n.d. | n.d. | 0.9 | n.d. | 1.2 | n.d. | 0.9 |
| 30°C/65%RH upright | 1.1 | n.d. | n.d. | n.d. | 0.8 | n.d. | 0.9 | n.d. | 0.9 |
| 30°C/65%RH inverted | 1.1 | n.d. | n.d. | n.d. | 0.9 | n.d. | 0.9 | n.d. | 0.9 |

From the above data it can be observed that with for all samples, the values are within the preferred range. This further indicates that the suspensions are completely chemically stable over a period of 36 months at 25 °C or 30 °C.

**Preservative efficacy for the pharmaceutical aqueous suspension**

Moreover, a preservative efficacy test (PET) was carried out using a combination of methyl paraben and propyl paraben. First, the preservative efficacy of the pharmaceutical aqueous suspension with 200 mg/mL flubendazole, but lacking methyl paraben and/or propyl paraben was analyzed. Test organisms included *P. aeruginosa, S. Aureus, E. coli, C. albicans* and *A. brasiliensis*. At day 14 and 28, the concentration of organism was measured according to methods known in the art such as described in Antimicrobial effectiveness testing, USP Chapter 51 and Scott et al., Journal of Pharmaceutical Science and Technology, 2002. For PET to provide a "pass" conclusive, the log10 of the counts per ml after 14 days should be at most 3. In addition there should be no increase (NI) in numbers of viable microorganism after 28 days compared to 14 days.

Table 7: Preservative efficacy test without preservatives (comparative data).

| Organism | PET conclusion |
|---|---|
| ***P. aeruginosa*** | Pass |
| **S. *Aureus*** | Fail |
| ***E. coli*** | Pass |
| ***C. albicans*** | Fail |
| ***A. brasiliensis*** | Fail |

The results show that without preservatives MPB or PPB, organisms such as *S. Aureus, C. albicans,* and *A. brasiliensis* readily grow in the suspension. The results of the PET without preservatives therefore result in an overall fail and would be unsuitable for use in drinking water.

Next, a PET for different concentrations of methyl paraben and/or propyl paraben (MPB, PPB, respectively) preservative according to the present invention was added to various batches of pharmaceutical aqueous suspension were analyzed. In addition, propylene glycol concentration was increased in a single instance and sodium chloride (NaCl) was omitted in another. PET was scored on a pass/fail criteria.

Table 8: PET of different concentrations preservatives in the suspension

| Sample | MPB % | PPB % | MPB+ PPB % | PG % | NaCl % | Conclusion |
|---|---|---|---|---|---|---|
| **#1** | 0.18 | 0.02 | 0.20 | 20 | 0.35 | Fail |
| **#2** | 0.18 | 0.02 | 0.20 | 20 | - | Fail |
| **#3** | 0.18 | 0.00 | 0.18 | 20 | 0.35 | Fail |
| **#4** | 0.00 | 0.00 | 0.00 | 20 | 0.35 | Fail |
| **#5** | 0.27 | 0.075 | 0.345 | 20 | 0.35 | Pass |
| **#6** | 0.38 | 0.11 | 0.49 | 30 | 0.35 | Pass |
| **#7** | 0.216 | 0.06 | 0.276 | 20 | - | Fail |
| **#8** | 0.216 | 0.06 | 0.276 | 20 | 0.35 | Pass |

The results show that samples #5 and #6 meet the acceptance criteria. In said batches, no increase (NI) was observed between day 28 and 14 and log10 counts for organisms was below 3. The combination batch #6 also meets the acceptance criteria for oral preparations but this high concentration of propylene glycol and preservatives is not required for preservation and the high amount of propylene glycol potentially could have a negative effect on stability. The lower limit of preservative efficacy for MPB and PPB was also tested (see samples #6, #7, and #8). The results show that the sodium chloride (present in #8 and absent in #7) has an impact on the preservation. The sample #7 without sodium chloride failed the preservative efficacy testing whereas the sample #8 having the same amount of preservatives with sodium chloride passed the test. This demonstrates the additional benefit for sodium chloride in the formulation.

(Concentrated) medicated water

**Materials**

The same materials as discussed above are used.

**Method for preparation**

Preparation of a (concentrated) medicated water can be carried out as followed. The pharmaceutical suspensions according to the present invention can be mixed with water and stirred in a way, e.g. manually stirring using a spoon, mechanical stirring using a whisk or mixer. The present inventors observed that within seconds of stirring the medicated water was homogeneous which was also determined by testing.

**Methods for measurements**

*Method for determining the amount of Flubendazole in the (concentrated) medicated water*

The identification of the active substance flubendazole in the (concentrated) medicated water is very similar to the measurement above for the pharmaceutical suspension.

For low medicated water a solution is prepared by pipetting 100 microliter of the pharmaceutical aqueous suspension prepared as shown above to 1000 ml of water in a volumetric flask and homogenized and directly transferred to a container. To determine the amount of flubendazole at the start, directly 5 ml was pipetted and added to a 10 ml volumetric flask, to this 2 ml of formic acid was added to dissolve flubendazole and this was immediately diluted to 10 ml by the solvent. For the samples after a certain amount of time 5 ml of the sample from the top or the bottom is pipetted and are each separately added to a 10 ml volumetric flask, to this 2 ml of formic acid was added to dissolve flubendazole and this was immediately diluted to 10 ml by the solvent.

For high medicated water a reference solution is prepared by pipetted 1.5 ml of the pharmaceutical aqueous suspension prepared as shown above to 1000 ml of water in a volumetric flask and homogenized and directly transferred to a container. To determine the amount of flubendazole at the start, directly 5 ml was pipetted and added to a 25 ml volumetric flask, to this 3 ml of formic acid was added to dissolve flubendazole and this was immediately diluted to 25 ml by the solvent. For the samples after a certain amount of time 5 ml of the sample from the top or the bottom is pipetted and are each separately added to a 25 ml volumetric flask, to this 3 ml of formic acid was added to dissolve flubendazole and this was immediately diluted to 25 ml by the solvent.

For the concentrated medicated water (stock suspension) a reference solution is prepared by pipetted 150 ml of the pharmaceutical aqueous suspension prepared as shown above to 1000 ml of water in a volumetric flask and homogenized and directly transferred to a container. To determine the amount of flubendazole at the start, directly 100 microliter was pipetted and added to a 50 ml volumetric flask, to this 6 ml of formic acid was added to dissolve flubendazole and this was immediately diluted to 50 ml by the solvent. For the samples after a certain amount of time 5 ml of the sample from the top or the bottom is pipetted transferred to a small beaker, from which immediately 100 microliter is added to a 50 ml volumetric flask, to this 6 ml of formic acid was added to dissolve flubendazole and this was immediately diluted to 50 ml by the solvent.

A similar HPLC method is used, except that for the low medicated water a sample volume of 15 microliter is used instead of 5 microliter which is used for the high medicated water and the stock suspension.

The concentration of flubendazole in the sample of the (concentrated) medicated water is calculated by the following formula:

$$P = \frac{C_r x\, A_t x\, c}{A_r}\, x\, \frac{V_{flask}\, x\, \rho}{w_{pip}}\, x\, \frac{In_r}{In_t}$$

In which P is the concentration of flubendazole (mg/ml) in the sample solution, $C_r$ is the concentration of flubendazole (mg/ml) in the reference solution; At is the area under the curve of the peak of flubendazole of the sample solution and $A_r$ the area under the curve of the peak of flubendazole of the reference solution. $V_{flask}$ is the volume (ml) of the flask for the sample solution (as shown above), p the density of the suspension which are set to 1.000 g/ml for the low and high medicated water and to 1.011 g/ml for the stock suspension; $w_{pip}$ is the weight of the volume pipetted from the suspensions prepared, $In_r$ is the reference injection volume (5 microliter) and Int the sample injection volume (either 5 or 15 microliter).

*Method for determining the amount of (impurities/degradation products):*

This is carried out in the same manner as discussed above for the determination of the amount of flubendazole of the concentrated medicated water and the amounts are calculated as discussed above for the pharmaceutical aqueous suspension.

*Method for determining the particle size distribution of flubendazole:*

This is carried out using the method discussed above for the pharmaceutical aqueous suspension.

**Physical stability for the (concentrated) medicated water**

The physical stability of (concentrated) medicated water has been tested at several concentrations, being:

- 0.10 ml of the pharmaceutical aqueous suspension per liter of water
    - hereinafter low medicated water
    - comprising 20 mg flubendazole per liter medicated water
- 1.5 ml of pharmaceutical aqueous suspension per liter of water
    - hereinafter high medicated water
    - comprising 0.3 g (300 mg) flubendazole per liter medicated water
- 150 ml of pharmaceutical aqueous suspension per liter of water
    - o hereinafter concentrated medicated water or stock suspension
    - 100 x high therapeutic concentration for 1% use in dosing pump
    - o comprising 30 g of flubendazole per liter concentrated medicated water

Firstly, the concentration of flubendazole in the (concentrated) medicated water was analyzed after 0 and 24 hours (at the top and bottom of a 1L container). A significant variation in these values could indicate instability of the (concentrated) medicated water due to sedimentation of the solid flubendazole particles from the suspension. Values are determined after 24 hours at top and bottom of the container and are relative to values at 0 hours taken from a solution directly after mixing. The tests are carried out at 25 °C (± 2 °C) and 60 % RH (±5%) or at 30 °C (± 2 °C) and 65 % RH (±5%). Values of the concentration at top and bottom after 24 hours are the means of 2 to 4 observations per combination of hardness, concentration and condition.

Soft water/low pH is water having a pH range from 5.0 to 7.0 and 60 mg/L or less of calcium carbonate. Hard water/high pH is water having a pH range from 8.0 to 9.0 and 180 to 350 mg/L of calcium carbonate.

Table 9. stability against sedimentation of (concentrated) medicated water.

| Hardness | Concentr. (mL/L) | Conditions | At 0h | At top after 24h | At bottom after 24h |
|---|---|---|---|---|---|
| Soft | 0.1 | 25°C/60%RH | 100 % | 97 % | 98 % |
| Hard | 0.1 | 25°C/60%RH | 100 % | 98 % | 98 % |
| Soft | 1.5 | 25°C/60%RH | 100 % | 99 % | 99 % |
| Hard | 1.5 | 25°C/60%RH | 100 % | 99 % | 99 % |
| Soft | 1.5 | 30°C/65%RH | 100 % | 100 % | 99 % |
| Soft | 150 | 25°C/60%RH | 100 % | 101 % | 101 % |
| Hard | 150 | 25°C/60%RH | 100 % | 100 % | 99 % |

Results from this first analysis on the physical stability show that the relative concentrations are within the preferred range. The relative concentrations do not alter significantly after 24 hours, nor between top and bottom measurements. Results further show that type of water, concentration or storage condition do not affect the relative concentration after 24 hours, nor between top and bottom measurements. Thus, the (concentrated) medicated water

according to the invention is not prone to sedimentation under these conditions.

All concentrations of medicated water were tested for hard water and soft water. All suspensions were stored in a slightly open HDPE jar without stirring, to reflect the worst case scenario conditions in practice. After 24 hours storage at 25 °C (± 2 °C) or at 30 °C (± 2 °C), samples were taken near the top and at the bottom of the jar. As discussed above, the (concentrated) medicated water is considered to be stable against sedimentation if after storing the amount of flubendazole near the top as well as the amount of flubendazole at the bottom are both between 90% and 100% of the calculated (theoretical) amount of flubendazole. All of the above cited samples were tested for the amount of flubendazole and the results demonstrated that the amount of flubendazole near the top and at bottom after storage for 24 hours were within the required ranges. In addition, all samples meet the specification, with no visible segregation. This clearly shows physical stability against sedimentation (no difference between top and bottom of jar) after 24 hours.

For acceptable results of the (concentrated) medicated water according to the invention, the D50 particle size should be less than 0.45 micron and > 90 % of particles should be less than 1.0 micron (Table 10). These were tested for soft water only, all at 25 °C (± 2 °C).

Table 10. Stability against aggregation for (concentrated) medicated water; D50 value, and percentage of particles less than 1.0 micron

| Hardness | Concent. (mL/L) | D50 after 0 hour | D50 after 24 hours | %≤1.0 micron after 0 hour | %≤1.0 micron after 24 hours |
|---|---|---|---|---|---|
| Soft | 0.1 | NA | NA | NA | NA |
| Soft | 1.5 | 0.16 | 0.16 | 96% | 96% |
| Soft | 150 | 0.16 | 0.16 | 96% | 96% |
| *NA means data is not available because the concentration is too low to be determined by laser diffraction particle size analyzer.* | | | | | |

From the above data it can be observed that for all samples, the values are within the preferred range. In addition, the data clearly shows that the (concentrated) medicated water are completely physically stable against aggregation. Results further show no agglomeration or aggregation as particle size distribution values remain stable over time.

**Chemical stability for the (concentrated) medicated water**

After having tested the physical stability of the (concentrated) medicated water according to the invention, the chemical stability of flubendazole of the (concentrated) medicated water was tested. To this end, the samples as described above were analyzed for the concentration (mg/L) of flubendazole after storage for 24 hours (24h) and compared to the theoretical amount (0h). For all samples, the amount of flubendazole should be between 90 and 110% to be considered chemically stable. In addition, the amount of impurities derived from chemical degradation of flubendazole, being a measure of the chemical stability are provided. The amount of impurities should be less than 2 %.

Table 11. Concentration of flubendazole before and after storage of (concentrated) medicated water. Concentration in % compared to theoretical amount.

| Hardness | Conc. (mL/L) | Conditions | Conc. at 0h | Concentration after 24 h (top sample) | Concentration after 24 h (bottom sample) |
|---|---|---|---|---|---|
| Soft | 0.1 | 25°C/ 60%RH | 100% | 97 % | 98% |
| Hard | 0.1 | 25°C/ 60%RH | 100% | 98 % | 98% |
| Soft | 1.5 | 25°C/ 60%RH | 100% | 100 % | 99% |
| Hard | 1.5 | 25°C/ 60%RH | 100% | 99 % | 98% |
| Soft | 150 | 25°C/ 60%RH | 100% | 104 % | 102% |
| Hard | 150 | 25°C/ 60%RH | 100% | 103 % | 102% |

From the above data it is observed that for all samples, the values are within the preferred range. This indicates that the (concentrated)medicated waters are chemically stable.

Next, the amount of impurities and/or degradation products that form within the (concentrated) medicated waters was analyzed. Preferred is that the amount is less than or equal to 2 %.

Table 12. Amount of impurities/degradation products in pharmaceutical aqueous suspension. amounts in percentages per weight (wt.%).

| Hardness | Conc. (mL/L) | Conditions | Conc. at 0h | Concentration after 24 h (top sample) | Concentration after 24 h (bottom sample) |
|---|---|---|---|---|---|
| Soft | 0.1 | 25°C/ 60%RH | 1.1% | 1.2% | 1.2% |
| Hard | 0.1 | 25°C/ 60%RH | 1.2% | 1.2% | 1.1% |
| Soft | 1.5 | 25°C/ 60%RH | 0.9% | 0.8% | 1.0% |
| Hard | 1.5 | 25°C/ 60%RH | 1.0% | 0.9% | 0.9% |
| Soft | 150 | 25°C/ 60%RH | 1.0% | 1.0% | 1.1% |
| Hard | 150 | 25°C/ 60%RH | 1.0% | 1.0% | 0.9% |

From the above data it can be observed that with for all samples, the values are within the preferred range. This further indicates that the suspensions are chemically stable over a period of 24 hours.

## Claims

**1.** A pharmaceutical aqueous suspension comprising:

* at most 35 wt.% of flubendazole, based on the weight of the pharmaceutical aqueous suspension, as solid particles having a particle size distribution in which D50 ≤ 0.45 micrometer and ≥ 90 % of particles are ≤ 1.0 micrometer measured according to the method in the description;
* a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25,
* one or more preservatives, and
* water.

**2.** The pharmaceutical aqueous suspension according to claim 1, wherein the non-ionic triblock copolymer surfactant has a molecular weight of at least 9000 g/mole.

**3.** The pharmaceutical aqueous suspension according to claim 1 or 2, wherein the non-ionic triblock copolymer surfactant is present in an amount of between 2 and 10 wt.% based on the weight of the pharmaceutical aqueous suspension.

**4.** The pharmaceutical aqueous suspension according to any one of the preceding claims, wherein the poly(propylene oxide)-block has a molecular mass of between 2000 and 6000 gram/mole, preferably between 3000 and 5000 gram/mole.

**5.** The pharmaceutical aqueous suspension according to any one of the preceding claims, wherein the one or more preservatives are of the paraben-type, preferably wherein two preservatives of the paraben-type are present being methyl paraben and propyl paraben, more preferably in a ratio of between 10:1 and 1:1, and/or wherein said one or more preservatives are present in a total amount of between 0.1 and 0.6 wt.%, preferably between 0.2 and 0.5 wt.% based on the weight of the pharmaceutical aqueous suspension.

**6.** The pharmaceutical aqueous suspension according to any one of the preceding claims, further comprising an alkylene glycol as a water-soluble co-solvent, preferably wherein said alkylene glycol is propylene glycol (PG), preferably wherein said alkylene glycol is present in an amount of between 15 and 25 wt.%, more preferably between 17 and 21 wt.% based on the weight of the pharmaceutical aqueous suspension.

**7.** The pharmaceutical aqueous suspension according to any one of the preceding claims, wherein water is present in an amount of between 20 and 70 wt.% based on the weight of the pharmaceutical aqueous suspension.

**8.** The pharmaceutical aqueous suspension according to any one of the preceding claims, further comprising sodium chloride in an amount of between 0.25 and 0.6 wt.%, preferably between 0.35 and 0.5 wt.% based on the weight of the pharmaceutical aqueous suspension.

**9.** The pharmaceutical aqueous suspension according to any one of the preceding claims, having a stability against aggregation of at least 36 months at 25 °C and/or being resuspensible in 30 seconds after storing for at least 36 months at 25 °C.

**10.** The pharmaceutical aqueous suspension according to any one of the preceding claims being an aqueous suspension of:

* between 40 and 60 wt.%, based on the weight of the pharmaceutical aqueous suspension, of water;
* between 15 and 25 wt.%, based on the weight of the pharmaceutical aqueous suspension, of propylene glycol;
* between 15 and 25 wt.%, based on the weight of the pharmaceutical aqueous suspension, of solid flubendazole particles having a particle size distribution in which D50 ≤ 0.45 micrometer and ≥ 90 % of particles are ≤ 1.0 micrometer;
* between 2 and 6 wt.% based on the weight of the pharmaceutical aqueous suspension of poloxamer 407 having a molecular mass of the poly(propylene oxide)-block of 4000 gram/mole and comprising a combined weight of said polyoxyethylene-blocks of at 70 wt.% based on the total weight of the copolymer surfactant;
* in total between 0.1 and 1.0 wt.%, based on the weight of the pharmaceutical aqueous suspension, of two paraben preservatives, being methyl paraben and propyl paraben; and
* between 0.1 and 1.0 wt.% based on the weight of the pharmaceutical aqueous suspension of sodium chloride;
* said pharmaceutical aqueous suspension being physically and chemically stable for at least 36 months at 25 °C.

**11.** A medicated water comprising water and the pharmaceutical aqueous suspension according to any one of the preceding claims in an amount of between 0.1 ml and 1.5 ml per liter of water, preferably having a stability against sedimentation of at 24 hours at 25 °C.

**12.** A concentrated medicated water comprising water and the pharmaceutical aqueous suspension according to any one of claims 1-10 in an amount of between 50 and 300 ml, preferably having a stability against sedimentation of at 24 hours at 25 °C.

**13.** A process of preparing a pharmaceutical aqueous suspension, comprising the steps of:

* preparing a mixture of solid flubendazole particles, a non-ionic triblock copolymer surfactant comprising a central poly(propylene oxide)-block having at least 30 propylene oxide (PO) units, flanked by two poly(ethylene oxide)-blocks, each having at a certain number of ethylene oxide (EO) units, wherein the ratio of number of PO units to total number of EO units is at least 0.2, preferably at least 0.25, and water;
* milling said mixture so obtain an aqueous suspension; and
* adding one or more preservatives to said aqueous suspension;

in order to obtain a pharmaceutical aqueous suspension composition comprising at most 35 wt.% of flubendazole, based on the weight of the pharmaceutical aqueous suspension, as solid particles having a particle size distribution in which D50 ≤ 0.45 micrometer and ≥ 90 % of particles are ≤ 1.0 micrometer.

**14.** The pharmaceutical aqueous suspension according to any one of claims 1-10 for use in a method for the treatment of internal parasites in mammals, such as poultry and pigs or for the preparation of a medicated water to provide a dosage of between 1 and 2.5 mg of flubendazole per kg of body weight per day.

## Patentansprüche

**1.** Pharmazeutische wässrige Suspension, umfassend:

* höchstens 35 Gew.-% Flubendazol, bezogen auf das Gewicht der pharmazeutischen wässrigen Suspension, als feste Partikel mit einer Teilchengrößenverteilung, bei der D50 ≤ 0,45 Mikrometer und ≥ 90 % der Partikel ≤ 1,0 Mikrometer sind, gemessen gemäß dem Verfahren in der Beschreibung;
* ein nichtionisches Triblock-Copolymer-Tensid, umfassend einen zentralen Poly(propylenoxid)-Block mit mindestens 30 Propylenoxid(PO)-Einheiten, flankiert von zwei Poly(ethylenoxid)-Blöcken, die jeweils eine bestimmten Anzahl von Ethylenoxid(EO)-Einheiten aufweisen, wobei das Verhältnis der Anzahl von PO-Einheiten zur Gesamtzahl von EO-Einheiten mindestens 0,2, vorzugsweise mindestens 0,25, beträgt,
* einen oder mehrere Konservierungsstoffe und
* Wasser.

2. Pharmazeutische wässrige Suspension nach Anspruch 1, wobei das nichtionische Triblock-Copolymer-Tensid ein Molekulargewicht von mindestens 9000 g/mol aufweist.

3. Pharmazeutische wässrige Suspension nach Anspruch 1 oder 2, wobei das nichtionische Triblock-Copolymer-Tensid in einer Menge zwischen 2 und 10 Gew.- %, bezogen auf das Gewicht der pharmazeutischen wässrigen Suspension, vorliegt.

4. Pharmazeutische wässrige Suspension nach einem der vorhergehenden Ansprüche, wobei der Poly(propylenoxid)-Block eine Molekülmasse zwischen 2000 und 6000 Gramm/mol, vorzugsweise zwischen 3000 und 5000 Gramm/mol, aufweist.

5. Pharmazeutische wässrige Suspension nach einem der vorhergehenden Ansprüche, wobei das eine oder die mehreren Konservierungsstoffe vom Paraben-Typ sind, wobei vorzugsweise zwei Konservierungsstoffe vom Paraben-Typ vorliegen, bei denen es sich um Methylparaben und Propylparaben handelt, besonders bevorzugt in einem Verhältnis zwischen 10:1 und 1:1, und/oder wobei der eine oder die mehreren Konservierungsstoffe in einer Gesamtmenge zwischen 0,1 und 0,6 Gew.-%, vorzugsweise zwischen 0,2 und 0,5 Gew.-%, bezogen auf das Gewicht der pharmazeutischen wässrigen Suspension, vorliegen.

6. Pharmazeutische wässrige Suspension nach einem der vorhergehenden Ansprüche, weiterhin umfassend ein Alkylenglykol als wasserlösliches Cosolvens, wobei es sich bei dem Alkylenglykol vorzugsweise um Propylenglykol (PG) handelt, wobei das Alkylenglykol vorzugsweise in einer Menge zwischen 15 und 25 Gew.-%, besonders bevorzugt zwischen 17 und 21 Gew.-%, bezogen auf das Gewicht der pharmazeutischen wässrigen Suspension, vorliegt.

7. Pharmazeutische wässrige Suspension nach einem der vorhergehenden Ansprüche, wobei Wasser in einer Menge zwischen 20 und 70 Gew.- %, bezogen auf das Gewicht der pharmazeutischen wässrigen Suspension, vorliegt.

8. Pharmazeutische wässrige Suspension nach einem der vorhergehenden Ansprüche, weiterhin umfassend Natriumchlorid in einer Menge zwischen 0,25 und 0,6 Gew.-%, vorzugsweise zwischen 0,35 und 0,5 Gew.-%, bezogen auf das Gewicht der pharmazeutischen wässrigen Suspension.

9. Pharmazeutische wässrige Suspension nach einem der vorhergehenden Ansprüche, die eine Aggregationsstabilität von mindestens 36 Monaten bei 25 °C aufweist und/oder nach mindestens 36-monatiger Lagerung bei 25 °C innerhalb von 30 Sekunden resuspendierbar ist.

10. Pharmazeutische wässrige Suspension nach einem der vorhergehenden Ansprüche, bei der es sich um eine wässrige Suspension aus Folgendem handelt:

* zwischen 40 und 60 Gew.-%, bezogen auf das Gewicht der pharmazeutischen wässrigen Suspension, an Wasser;
* zwischen 15 und 25 Gew.-%, bezogen auf das Gewicht der pharmazeutischen wässrigen Suspension, an Propylenglykol;
* zwischen 15 und 25 Gew.-%, bezogen auf das Gewicht der pharmazeutischen wässrigen Suspension, an festen Flubendazolpartikeln mit einer Teilchengrößenverteilung, bei der D50 ≤ 0,45 Mikrometer und ≥ 90 % der Partikel ≤ 1,0 Mikrometer sind;
* zwischen 2 und 6 Gew.-%, bezogen auf das Gewicht der pharmazeutischen wässrigen Suspension, an Poloxamer 407 mit einer Molmasse des Poly(propylenoxid)-Blocks von 4000 Gramm/mol, und umfassend ein kombiniertes Gewicht der Polyoxyethylen-Blöcke von bei 70 Gew.-%, bezogen auf das Gesamtgewicht des Copolymer-Tensids;
* insgesamt zwischen 0,1 und 1,0 Gew.-%, bezogen auf das Gewicht der pharmazeutischen wässrigen Suspension, zweier Parabenkonservierungsstoffe, nämlich Methylparaben und Propylparaben; und
* zwischen 0,1 und 1,0 Gew.-%, bezogen auf das Gewicht der pharmazeutischen wässrigen Suspension, an Natriumchlorid;

* wobei die pharmazeutische wässrige Suspension bei 25 °C mindestens 36 Monate physikalisch und chemisch stabil ist.

11. Medizinisches Wasser, umfassend Wasser und die pharmazeutische wässrige Suspension nach einem der vorhergehenden Ansprüche in einer Menge zwischen 0,1 ml und 1,5 ml pro Liter Wasser, vorzugsweise mit einer Sedimentationsstabilität von 24 Stunden bei 25 °C.

12. Konzentriertes medizinisches Wasser, umfassend Wasser und die pharmazeutische wässrige Suspension nach einem der Ansprüche 1-10 in einer Menge zwischen 50 und 300 ml, vorzugsweise mit einer Sedimentationsstabilität von 24 Stunden bei 25 °C.

13. Verfahren zur Herstellung einer pharmazeutischen wässrigen Suspension, welches die folgenden Schritte umfasst:

* die Zubereitung einer Mischung aus festen Flubendazolpartikeln, einem nichtionischen Triblock-Copolymer-Tensid, umfassend einen zentralen Poly(propylenoxid)-Block mit mindestens 30 Propylenoxid(PO)-Einheiten, flankiert von zwei Poly(ethylenoxid)-Blöcken, die jeweils eine bestimmten Anzahl von Ethylenoxid(EO)-Einheiten aufweisen, wobei das Verhältnis der Anzahl von PO-Einheiten zur Gesamtzahl von EO-Einheiten mindestens 0,2, vorzugsweise mindestens 0,25, beträgt, und Wasser;
* das Mahlen der Mischung unter Erhalt einer wässrigen Suspension; und
* das Hinzufügen eines oder mehrerer Konservierungsstoffe zu der wässrigen Suspension;
unter Erhalt einer pharmazeutischen wässrigen Suspensionszusammensetzung, die höchstens 35 Gew.-% Flubendazol, bezogen auf das Gewicht der pharmazeutischen wässrigen Suspension, als feste Partikel mit einer Teilchengrößenverteilung, bei der D50 ≤ 0,45 Mikrometer und ≥ 90 % der Partikel ≤ 1,0 Mikrometer sind.

14. Pharmazeutische wässrige Suspension nach einem der Ansprüche 1-10 zur Verwendung in einem Verfahren zur Behandlung von inneren Parasiten bei Säugetieren, wie Geflügel und Schweinen, oder zur Herstellung eines medizinischen Wassers, zur Bereitstellung einer Dosierung von 1 bis 2,5 mg Flubendazol pro kg Körpergewicht pro Tag.

**Revendications**

1. Suspension pharmaceutique aqueuse comprenant :

* au plus 35% en poids de flubendazole, par rapport au poids de la suspension pharmaceutique aqueuse, sous forme de particules solides ayant une distribution granulométrique dans laquelle D50 ≤ 0,45 micromètre, et ≥ 90% des particules sont ≤ 1,0 micromètre, mesuré selon le procédé décrit dans la description ;
* un tensioactif copolymère tribloc non ionique comprenant un bloc central de poly(oxyde de propylène) ayant au moins 30 unités d'oxyde de propylène (PO), flanqué de deux blocs de poly(oxyde d'éthylène), chacun ayant un certain nombre d'unités d'oxyde d'éthylène (EO), dans lequel le rapport entre le nombre d'unités PO et le nombre total d'unités EO est d'au moins 0,2, de préférence d'au moins 0,25,
* un ou plusieurs conservateurs, et
* de l'eau.

2. Suspension pharmaceutique aqueuse selon la revendication 1, dans laquelle le tensioactif copolymère tribloc non ionique a un poids moléculaire d'au moins 9000 g/mole.

3. Suspension pharmaceutique aqueuse selon la revendication 1 ou 2, dans laquelle le tensioactif copolymère tribloc non ionique est présent en une quantité comprise entre 2 et 10% en poids par rapport au poids de la suspension pharmaceutique aqueuse.

4. Suspension pharmaceutique aqueuse selon l'une quelconque des revendications précédentes, dans laquelle le bloc de poly(oxyde de propylène) a une masse moléculaire comprise entre 2000 et 6000 grammes/mole, de préférence entre 3000 et 5000 grammes/mole.

5. Suspension pharmaceutique aqueuse selon l'une quelconque des revendications précédentes, dans laquelle le ou les plusieurs conservateurs sont du type parabène, de préférence dans laquelle deux conservateurs du type parabène sont présents, à savoir le méthylparabène et le propylparabène, de préférence dans un rapport compris

entre 10:1 et 1:1, et/ou dans laquelle ledit un ou lesdits plusieurs conservateurs sont présents en une quantité totale comprise entre 0,1 et 0,6% en poids, de préférence entre 0,2 et 0,5% en poids par rapport au poids de la suspension pharmaceutique aqueuse.

**6.** Suspension pharmaceutique aqueuse selon l'une quelconque des revendications précédentes, comprenant en outre un alkylène glycol sous forme de cosolvant hydrosoluble, de préférence dans lequel ledit alkylène glycol est le propylène glycol (PG), de préférence dans lequel ledit alkylène glycol est présent en une quantité comprise entre 15 et 25% en poids, de préférence entre 17 et 21% en poids par rapport au poids de la suspension pharmaceutique aqueuse.

**7.** Suspension pharmaceutique aqueuse selon l'une quelconque des revendications précédentes, dans laquelle l'eau est présente en une quantité comprise entre 20 et 70% en poids par rapport au poids de la suspension pharmaceutique aqueuse.

**8.** Suspension pharmaceutique aqueuse selon l'une quelconque des revendications précédentes, comprenant en outre du chlorure de sodium en une quantité comprise entre 0,25 et 0,6% en poids, de préférence entre 0,35 et 0,5% en poids par rapport au poids de la suspension pharmaceutique aqueuse.

**9.** Suspension pharmaceutique aqueuse selon l'une quelconque des revendications précédentes, ayant une stabilité à l'agrégation d'au moins 36 mois à 25°C et/ou pouvant être remise en suspension en 30 secondes après une conservation pendant au moins 36 mois à 25°C.

**10.** Suspension pharmaceutique aqueuse selon l'une quelconque des revendications précédentes, étant une suspension aqueuse de :

* entre 40 et 60% en poids, par rapport au poids de la suspension pharmaceutique aqueuse, d'eau ;
* entre 15 et 25% en poids, par rapport au poids de la suspension pharmaceutique aqueuse, de propylène glycol ;
* entre 15 et 25% en poids, par rapport au poids de la suspension pharmaceutique aqueuse, de particules solides de flubendazole ayant une distribution granulométrique dans laquelle D50 ≤ 0,45 micromètre, et ≥ 90% des particules sont ≤ 1,0 micromètre ;
* entre 2 et 6% en poids, par rapport au poids de la suspension pharmaceutique aqueuse, de poloxamère 407 ayant une masse moléculaire du bloc de poly(oxyde de propylène) de 4000 grammes/mole et comprenant un poids combiné desdits blocs de poly(oxyde d'éthylène) de 70% en poids par rapport au poids total du tensioactif copolymère ;
* au total entre 0,1 et 1,0% en poids, par rapport au poids de la suspension pharmaceutique aqueuse, de deux conservateurs parabènes, à savoir le méthylparabène et le propylparabène ; et
* entre 0,1 et 1,0% en poids, par rapport au poids de la suspension pharmaceutique aqueuse, de chlorure de sodium ;
* ladite suspension pharmaceutique aqueuse étant physiquement et chimiquement stable pendant au moins 36 mois à 25°C.

**11.** Eau médicinale comprenant de l'eau et la suspension pharmaceutique aqueuse selon l'une quelconque des revendications précédentes en une quantité comprise entre 0,1 ml et 1,5 ml par litre d'eau, ayant de préférence une stabilité à la sédimentation de 24 heures à 25°C.

**12.** Eau médicinale concentrée comprenant de l'eau et la suspension pharmaceutique aqueuse selon l'une quelconque des revendications 1 à 10 en une quantité comprise entre 50 et 300 ml, ayant de préférence une stabilité à la sédimentation de 24 heures à 25°C.

**13.** Procédé de préparation d'une suspension pharmaceutique aqueuse, comprenant les étapes de :

* préparation d'un mélange de particules solides de flubendazole, d'un tensioactif copolymère tribloc non ionique comprenant un bloc central de poly(oxyde de propylène) ayant au moins 30 unités d'oxyde de propylène (PO), flanqué de deux blocs de poly(oxyde d'éthylène), chacun ayant un certain nombre d'unités d'oxyde d'éthylène (EO), dans lequel le rapport entre le nombre d'unités PO et le nombre total d'unités EO est d'au moins 0,2, de préférence d'au moins 0,25, et d'eau ;
* broyage dudit mélange pour obtenir une suspension aqueuse ; et
* ajout d'un ou plusieurs conservateurs à ladite suspension aqueuse ;

afin d'obtenir une composition de suspension pharmaceutique aqueuse comprenant au plus 35% en poids de flubendazole, par rapport au poids de la suspension pharmaceutique aqueuse, sous forme de particules solides ayant une distribution granulométrique dans laquelle D50 $\leq$ 0,45 micromètre, et $\geq$ 90% des particules sont $\leq$ 1,0 micromètre.

**14.** Suspension pharmaceutique aqueuse selon l'une quelconque des revendications 1 à 10, pour une utilisation dans un procédé permettant le traitement des parasites internes chez les mammifères, comme les volailles et les porcs, ou la préparation d'une eau médicinale pour fournir une dose comprise entre 1 et 2,5 mg de flubendazole par kg de poids corporel par jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007144362 A1
- BR 102018017054


### Non-patent literature cited in the description


- *CHEMICAL ABSTRACTS*, 31430-15-6
- Antimicrobial effectiveness testing. USP
- **SCOTT et al.** *Journal of Pharmaceutical Science and Technology*, 2002